(19) Europäisches Patentamt
European Patent Office
Office européen des brevets

(11) Veröffentlichungsnummer: **0 310 012**
**A2**

(12) # EUROPÄISCHE PATENTANMELDUNG

(21) Anmeldenummer: **88115955.2**

(22) Anmeldetag: **28.09.88**

(51) Int. Cl.⁴: **C07C 99/12 , C07C 101/28 , C07B 57/00**

(30) Priorität: **01.10.87 DE 3733222**

(43) Veröffentlichungstag der Anmeldung: **05.04.89 Patentblatt 89/14**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Keller, Reinhold, Dr.**
**Kaunweg 8**
**D-6232 Bad Soden am Taunus(DE)**
Erfinder: **Müller, Wolfgang, Dr.**
**Theodor-Körner-Strasse 12**
**D-6238 Hofheim am Taunus(DE)**

(54) **Verfahren zur Herstellung von optisch reinem Homophenylalanin.**

(57) Optisch reine Homophenylalanine können hergestellt werden, indem die entsprechenden racemischen Ester einer selektiven Hydrolyse mit Subtilisin unterworfen werden. Dabei wird der L-Carbonsäureester gespalten. Die D-Verbindung unterliegt dieser Hydrolyse nicht und kann auf diese Weise leicht von der L-Verbindung abgetrennt werden.

**EP 0 310 012 A2**

Xerox Copy Centre

## Verfahren zur Herstellung von optisch reinem Homophenylalanin

Die Erfindung betrifft ein Verfahren zur Herstellung von optisch reinem L- bzw. D-Homophenylalanin. das die Synthese von N- und O-geschütztem racemischem Homophenylalanin sowie dessen enzymatische Spaltung mittels Subtilisin umfaßt.

L-Homophenylalanin kann beispielsweise zur Synthese einer Ramipril-Vorstufe dienen. Ramipril (2-{N-[-(S)-1-Ethoxycarbonyl-3-phenylpropyl]-L-alanyl}-(1S,3S,5S)-2-azabicyclo[3.3.0]octan-3-carbonsäure)

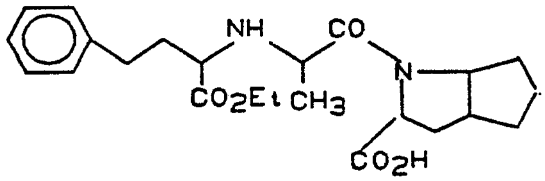

ist ein hochwirksamer ACE-Hemmer.

Enzymatische Racematspaltungen von proteinogenen Aminosäuren sind bereits beschrieben.

Aus der deutschen Offenlegungsschrift 2 215 853 ist es bekannt, racemische, ringsubstituierte Phenylalanine in die entsprechenden L-Aminosäuren dadurch zu überführen, daß man das Racemat zunächst mit einem Alkanol mit bis zu 4 Kohlenstoffatomen verestert, den racemischen Ester der Einwirkung eines Chymotrypsin bei einem sauren pH unterwirft, die L-Aminosäure durch Fällung isoliert und gegebenenfalls den Ester der D-Aminosäure aus dem Filtrat extrahiert und verseift. Als Enzym wird das α-Chymotrypsin bevorzugt; der bevorzugte pH-Bereich bei der enzymatischen Spaltung liegt bei 5 bis 6. Die nach Abtrennung der L-Aminosäure verbliebene Flüssigkeit wird vor der Extraktion des Esters der D-Aminosäure alkalisch gestellt.

In der deutschen Offenlegungsschrift 34 38 189 wird ein Verfahren zur Herstellung von aromatisch substituierten L-Aminosäuren durch Spaltung der racemischen niederen Alkylester mit α-Chymotrypsin im schwach sauren wäßrigen Medium beschrieben, bei dem das Reaktionsgemisch schwach alkalisch gestellt wird, der Ester der D-Aminosäure mit einem organischen Lösungsmittel extrahiert, der Ester racemisiert und wieder in den Prozeß zurückgeführt wird, und die L-Aminosäure aus dem wäßrigen alkalischen Extrakt nach Ansäuern isoliert wird.

In der deutschen Offenlegungsschrift 29 27 534 sind optisch reine heterocyclische α-Aminosäuren beschrieben sowie ein Verfahren zur Herstellung dieser Verbindungen, das auf der Umsetzung eines racemischen α-Aminosäurederivats mit einem trägergebundenen, proteolytischen Enzym beruht. Als verwendbare Enzyme werden unter anderem Subtilisin und α-Chymotrypsin beschrieben.

In Chem. Ber. 114, 173-189 (1981) ist die Darstellung von bestimmten Aminosäuren wie Phenylalanin, Glycin, Alanin, Leucin oder Valin aus Halogencarbonsäurealkylestern mit Alkalimetallcyanaten beschrieben.

Überraschend wurde nun gefunden, daß racemisches N- und O-geschütztes Homophenylalanin - ein Derivat einer nichtproteinogenen Aminosäure - mit Subtilisin enantioselektiv gespalten werden kann. Dies ist um so überraschender, als bisher angenommen wurde, daß nur proteinogene oder allenfalls leicht modifizierte proteinogene Aminosäuren wie Phenylalanin oder phenylringsubstituierte Phenylalanine mit Subtilisin gespalten werden können. Unerwartet war ferner, daß sich amidgeschütztes Homophenylalanin mittels Subtilisin spalten läßt.

In Kombination mit dem wirtschaftlichen Herstellungsverfahren des N- und O-geschützten Homophenylalanin (s. loc. cit. Chem. Ber.), bietet die enzymatische Racematspaltung einen äußerst einfachen und preisgünstigen Zugang zu optisch reinem L- und D-Homophenylalanin.

Die Erfindung betrifft nunmehr:

Ein Verfahren zur Herstellung von optisch reinem Homophenylalanin, das dadurch gekennzeichnet ist, daß die racemische Verbindung der allgemeinen Formel I,

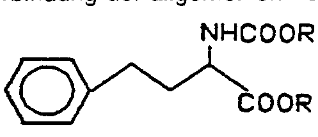

(I)

in der
R and R′ gleich oder verschieden sind und ($C_1$-$C_4$)-Alkyl bedeuten,
der Einwirkung von Subtilisin bei schwach saurem bis schwach alkalischem pH unterworfen wird.

Außerdem betrifft die Erfindung die Verwendung von L-Homophenylalanin zur Herstellung von Ramipril.

Als Schutzgruppen für die Amino- und Carboxyfunktion kommen prinzipiell solche in Betracht, die auch in der Peptidchemie beim Aufbau von Peptiden Verwendung finden.

Als Amino-Schutzgruppen kommen bevorzugt lineare oder gegebenenfalls verzweigte Carbonyloxyalkyle mit 1-4 Kohlenstoffatomen, insbesondere der Acetylrest in Betracht. Bevorzugte Carboxy Schutzgruppen sind lineare oder verzweigte Alkyle mit 1-4 Kohlenstoffatomen, insbesondere Methyl und Ethyl, oder die Benzylgruppe (Carbonsäureester).

Die racemische Verbindung der Formel I ist auf einfache Weise erhältlich. So kann beispielsweise β-Phenyl-ethylbromid mit Malonsäurediethylester (R = Ethyl) zu 2-Ethoxycarbonyl-4-phenyl-buttersäureethylester umgesetzt werden, wonach anschließend mit einer starken Base wie KOH die Esterfunktion in 2-Stellung verseift wird, dann mit Brom in 2-Stellung bromiert und nach Protonierung durch Erhitzen decarboxyliert wird, wobei man den 2-Brom-4-phenyl-buttersäureethylester erhält.

Die Umsetzung dieses α-Halogen-carbonsäurealkylesters mit Alkalimetallcyanaten in Gegenwart von Alkoholen in dipolar aprotischen Lösungsmitteln erfolgt dann analog zu dem in Chem. Ber. 114, 173-189 (1981) beschriebenen Verfahren, wobei man die N-alkoxycarbonyl- und O-alkylgeschützten Homophenylalanine der Formel I erhält.

Die racemische Spaltung dieser geschützten Homophenylalanine beruht auf der selektiven Hydrolyse des L-Carbonsäureesters mittels Subtilisin (EC 3.4.4.16). Da die D-Verbindung dieser Hydrolyse nicht unterliegt, kann sie auf diese Weise leicht von der L-Verbindung abgetrennt werden.

Die enzymatische Spaltung erfolgt vorzugsweise bei einer Temperatur von 30-55° C, insbesondere bei 43-47° C, in einem pH-Bereich, der von schwach sauer bis schwach alkalisch variieren kann. Bevorzugt ist ein pH-Bereich von 5,5-7,5, insbesondere arbeitet man bei einem pH-Wert von 7,0. Das Substrat, die racemische Verbindung der Formel I, wird als 15-25 Gew.-%ige, bevorzugt 19-21 Gew.-%ige Lösung mit dem 3-5-fachen, bevorzugt 4-fachen Volumen an Wasser versetzt und anschließend mit 0,01-0,1 AU, bevorzugt 0,05-0,08 AU Subtilisin pro mmol (®Maxatase, Hersteller: Gistbrocades N.V. Delft/Niederlande; ®Optimase, Hersteller: Miles-Kali-Chemie, Hannover; ®Alcalase 2.5 L, Hersteller: Novo Industri AS, Kopenhagen/Dänemark) inkubiert und während der enzymatischen Umsetzung intensiv gerührt [Anson Units (AU) Novo-Standardtest mit Dimethylcasein bei 50° C und pH 8,3 (Firmenschrift)]. Der Verlauf und der Endpunkt der Reaktion kann durch pH-Wert-Messung bzw. durch die notwendige Neutralisation der entstehenden $H^+$-Ionen bestimmt werden. Die Neutralisation bzw. die Einhaltung des pH-Werts in den angegebenen Grenzen erfolgt durch Basenzusatz.

Das Substrat - die Verbindung der Formel I - kann beispielsweise in Wasser, Ethanol, Methanol oder beliebigen Wasser-Alkohol-Mischungen, bevorzugt jedoch in einem mit Wasser nicht oder nur begrenzt mischbaren Lösungsmittel wie Methylisobutylketon (MIBK) oder Essigester gelöst werden (zweiphasiges System).

Andererseits bietet die Suspension von N- und O-geschütztem Homophenylalanin allein in Wasser den Vorteil, daß auf Grund der unterschiedlichen Löslichkeitsprodukte von a) verestertem Homophenylalanin und b) von hydrolysiertem Homophenylalanin eine leichtere Abtrennung der gewünschten optischen Antipoden erfolgen kann.

Das Enzym Subtilisin kann sowohl als solches, als auch gebunden an einen Träger, eingesetzt werden (immobilisiertes Enzym). Als Enzymträger kommen beispielsweise anorganische Träger wie Aluminiumsilikate aber auch Polymere, organische Träger wie Cellulosen, modifizierte Polyacrylamidgele, die Amino- oder Hydroxylgruppen besitzen oder auch organische Copolymerisate aus Acrylamid, Methacrylaten oder Methacrylamid und Maleinsäureanhydrid in Frage. Die Herstellung entsprechender trägergebundener Enzyme ist beispielsweise beschrieben in Halwachs et al., Biotechnology and Bioengineering XIX (1977) 1667-1677 (Fixierung auf Aluminiumsilikat) oder DE-OS 29 27 534 (Fixierung auf Cellulose).
Bevorzugt wird Maxatase als solche, d.h. als wäßrige Lösung eingesetzt. Maxatase stellt eine rohe und preiswerte Subtilisin-Qualität für Waschmittelzwecke dar.

Zur Aufarbeitung der nach der enzymatischen Spaltung erhaltenen wäßrigen Lösung bieten sich beispielsweise die folgenden Möglichkeiten an:
Für den Fall, daß neben der L-Aminosäure auch die D-Aminosäure rein dargestellt werden soll, kann zunächst der nicht hydrolysierte D-Ester bei neutralem pH-Wert mit einem geeigneten organischen Lösungsmittel extrahiert und anschließend verseift werden. Als Lösungsmittel bieten sich beispielsweise Hexan, Toluol, Xylol, Essigsäureethylester oder Ether wie Diethylether oder Diisopropylether oder chlorierte Kohlenwasserstoffe wie Methylenchlorid, Chloroform oder Tetrachlorkohlenstoff an. Die L-Aminosäure wird

aus dem wäßrigen Rückstand durch Ansäuern isoliert. Je nach Löslichkeit der freien L-Säure kann diese unmittelbar oder nach Konzentration auskristallisieren. Die L-Säure kann aber auch in bekannter Weise durch Extraktion mit einem geeigneten Lösungsmittel isoliert werden. Die Reinigung erfolgt in bekannter Weise.

In den meisten Fällen kann jedoch auf eine Reindarstellung der D-Aminosäureester bzw. der D-Aminosäuren verzichtet werden. In diesem Fall ist es zweckmäßig, die D-Aminosäureester zu extrahieren, erneut zu racemisieren und in den enzymatischen Spaltprozeß zurückzuführen. Hierzu wird die nach der enzymatischen Spaltung erhaltene wäßrige Lösung der L-Aminosäure und des Esters der D-Aminosäure schwach alkalisch gestellt, vorteilhaft auf einen pH-Wert im Bereich von 7,5 bis 8, und - zweckmäßig bei Raumtemperatur - mit einem geeigneten organischen Lösemittel extrahiert, wobei die L-Aminosäure in der schwach alkalischen wäßrigen Phase zurückbleibt.

Als organisches Lösemittel für die Extraktion wählt man zweckmäßig ein solches, das wenig mit Wasser mischbar ist, da dann die Racemisierung besonders vorteilhaft ohne Isolierung des Esters erfolgen kann. Bevorzugt ist deshalb ein Lösemittel, das mit Wasser ein Azeotrop bildet und hierdurch eine leichte Trocknung des Extraktes erlaubt. Es ist daher aber auch möglich, wenn auch im allgemeinen nicht vorteilhaft, das zur Extraktion benutzte Lösemittel völlig abzutrennen und den Ester in Substanz zu racemisieren.

Geeignete Lösemittel für die Extraktion sind aliphatische Kohlenwasserstoffe wie Hexan, insbesondere aromatische Kohlenwasserstoffe wie Toluol oder Xylol, Ether wie Diethylether, Diisopropylether oder Di-n-butylether, Ketone wie Methylethylketon oder Methylisobutylketon, halogenierte Kohlenwasserstoffe wie Methylenchlorid, Chloroform oder Tetrachlormethan sowie Ester wie Ethylacetat oder Butylacetat.

Die Racemisierung erfolgt beispielsweise durch Kochen in Alkohol/Alkalimetallalkoholat. Bevorzugt erfolgt die Racemisierung in einem solchen Alkohol/Alkoholat, der auch Bestandteil der Amino- und/oder Carboxy-Schutzgruppe ist. Auf diese Weise führt die mit der Racematspaltung einhergehende Umesterung zu identischen Racematen. Die Konzentration der Alkalimetallalkoholate, bevorzugt Natriumalkoholate, beträgt 5-20 Gew.-%, bevorzugt 12-18 Gew.-%, bezogen auf den eingesetzten Alkohol. Die erneute Racemisierung der D-Ester, zwecks Rückführung in den enzymatischen Spaltprozess ist auch beschrieben in der Deutschen Patentanmeldung 3 438 189.

Aus dem schwach alkalischen, wäßrigen Rückstand, der die urethangeschützte L-Aminosäure enthält, wird beispielsweise durch Kochen in wäßriger Mischung aus Ameisensäure und Salzsäure die Urethangruppe hydrolysiert und anschließend das freie L-Aminosäure-Hydrochlorid isoliert. Die Reinigung kann in bekannter Weise erfolgen.

Man erhält L-Homophenylalanin in Ausbeuten von über 90 % und in einer optischen Reinheit von über 98 %. Durch die Kombination von enzymatischer Spaltung, Extraktion des D-Esters, dessen Racemisierung und Rückführung ist eine besonders zweckmäßige Verbindung dieser Arbeitsschritte gegeben, die auch die kontinuierliche Ausgestaltung des Verfahrens erlaubt.

Andererseits kann der urethangeschützte D,L-Homophenylalaninester auch in rein wäßrigem Medium trotz der sehr geringen Löslichkeit (ca. $6 \cdot 10^{-3}$%) enzymatisch enantioselektiv hydrolysiert werden. Durch den Wegfall des organischen Lösungsmittels treten keine Enzymdesaktivierungen auf und die Enantioselektivität wird erhöht.

In den folgenden Beispielen wird die Erfindung näher erläutert. Prozentangaben beziehen sich auf das Gewicht, sofern keine anderen Angaben gemacht sind.

**Beispiel 1a:**

Synthese von DL-2-Ethoxycarbonylamino-4-phenyl-buttersäureethylester (DL-Etoc-HPhe-OEt)

56,94 g (210 mmol) D,L-2-Brom-4-phenylbuttersäureethylester, 45 ml (735 mmol) abs. Ethanol, 210 ml abs. DMF und 25,53 g getrocknetes KOCN wurden 1 Stunde bei 100 °C in einem Einhalskolben gerührt. Nach dem Abkühlen (Eisbad) wird vom Salz abfiltriert und am Rotationsverdampfer (60 °C, Wasserbad) eingeengt. Nach Zugabe von 150 ml Aceton und erneutem abkühlen wurde das ausgefallene Restsalz wiederum abgetrennt. Die Lösung wurde eingeengt (Hochvakuum/50 °C) worauf beim Abkühlen nach Zugabe von Impfkristallen das braune Rohprodukt auskristallisierte. Das Rohprodukt wurde mit wenig kaltem Diethylether (-40 °C) verrieben und anschließend abgesaugt. Es verblieb ein gelblicher Festkörper. Die Mutterlauge wurde eingeengt und wie vorstehend beschrieben aufgearbeitet.

Ausbeute: 41,22 g (70 % d.Th.) Smp.: 65-66° C

Das Produkt kann durch Sublimation (Hochvakuum, Ölbad, ca. 120° C) in ein farbloses Reinprodukt überführt werden. Für die nachfolgend beschriebene Racematspaltung wurde jedoch das gelbliche Rohprodukt verwendet.

**Beispiel 1b:**

Enzymatische Racematspaltung von DL-2-Ethoxycarbonylamino-4-phenyl-buttersäureethylester (DL-Etoc-HPhe-OEt)

Variante 1: (Methylisobutylketon (MIBK)/$H_2O$-System)

8,38 g (30 mmol) Urethanester aus Beispiel 1a wurden in 40 ml MIBK gelöst, mit 160 ml $H_2O$ versetzt und der pH auf 7,00 einjustiert. Anschließend erfolgte die Zugabe von 1 g Alkalase (2.5 L) Lösung (2,5 AU) (Novo Industri) und die Titration mit 1 N NaOH bei 45° C unter kräftigem Rühren. Nach 5 Stunden erreichte man das Titrationsplateau (14,5 ml NaOH-Verbrauch). Es wurde abgekühlt und die Phasen getrennt. Die eingedampfte MIBK-Phase lieferte 4,01 g (95 % d.Th) D-Etoc-HPhe-OEt; ee ≧ 90 %. [1)]

Die $H_2O$-Phase wurde auf 50 ml Volumen eingeengt, gleiche Volumina Konz. HCl und Konz. Ameisensäure hinzugegeben und 16 Stunden unter Rückfluß gekocht. Das Hydrolysat wurde eingedampft. Ausbeute: 3,2 g (99 % d.Th.) L-H-Phe-OH x HCl ee ≧ 97 % [1)].

Durch Waschen mit Aceton kann das Produkt leicht entfärbt werden.

Variante 2: ($H_2O$-System)

5,58 g (20 mmol) Urethanester aus Beispiel 1a wurden in 200 ml $H_2O$, pH = 7,00 bei 30° C suspendiert. Nach Zugabe von 0,5 ml Alkalase 2.5 L Lösung (1,25 AU) erfolgt die Titration mit 0,5 N NaOH-Lösung.

Nach 6 ¾ Stunden waren 18,4 ml Lauge verbraucht und das Titrationsplateau erreicht.

Man kühlte auf 0° C, filtrierte ab und isolierte das Produkt:

Ausbeute 2,65 g (95 % d.Th.) D-Urethanester. Die wäßrige Phase enthielt im HPLC gerade noch nachweisbare Mengen an Ester und wurde 1 mal mit Essigester extrahiert.

(Die Verseifung der wäßrigen Phase kann wegen des vernachlässigbaren Gehalts an Ester auch ohne Extraktionsschritt durchgeführt werden. Es ergeben sich praktisch die identischen ee-Werte.)

Nach dieser Behandlung war die Wasserphase vollständig frei von Urethanester.

Die Lösung wurde auf 50 ml Volumen eingedampft und mit gleichen Volumina Konz. HCl und Konz. Ameisensäure versetzt. Nach 16 Stunden Kochen unter Rückfluß wurde eingeengt und isoliert. Ausbeute: 2,00 g (93 % d.Th.) L-Homophenylalanin x HCl ee ≧ 99 % [1)].

**Ansprüche**

1. Verfahren zur Herstellung von optisch reinem Homophenylalanin, dadurch gekennzeichnet, daß die racemische Verbindung der allgemeinen Formel I,

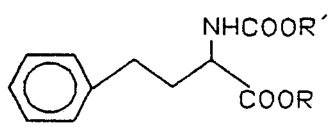

(I)

[1)] Bestimmung der ee-Werte nach pre column-Derivatisierung mit L-Boc-Cystein und ortho-Phthaldialdehyd der freien Aminosäuren (Methode nach R.H. Buck, Sandoz AG) mit RP-HPLC RP 18.

in der
R und R′ gleich oder verschieden sind und ($C_1$-$C_4$)-Alkyl bedeuten,
der Einwirkung von Subtilisin bei schwach saurem bis schwach alkalischem pH unterworfen wird.

2. Verfahren nach Anspruch 2, dadurch gekennzeichnet, daß die Umsetzung der Verbindung der allgemeinen Formel I mit Subtilisin in Gegenwart eines Lösungsmittels, ausgewählt aus Wasser, Ethanol, Methanol und den Mischungen Wasser/Methylisobutylketon, Wasser/Essigester, Wasser/Methanol und Wasser/Ethanol durchgeführt wird.

3. Verfahren nach Anspruch 1, dadurch gekennzeichnet, daß der pH-Wert 5,5 bis 7,5 beträgt.

4. Verfahren nach Anspruch 1, dadurch gekennzeichnet, daß die Umsetzung bei einer Reaktionstemperatur von 30-55 °C durchgeführt wird.

5. Verfahren nach Anspruch 1, dadurch gekennzeichnet, daß Subtilisin zu racemischer Verbindung im Verhältnis 0,01 bis 0,1 AU/mmol Substrat zugegeben wird.

6. Verwendung von L-Homophenylalanin zur Herstellung von Ramipril.